Europäisches Patentamt

European Patent Office

Office européen des brevets

(19)

(11) Publication number: **0 040 641**
**B1**

(12) EUROPEAN PATENT SPECIFICATION

(45) Date of publication of patent specification: **04.07.84**

(51) Int. Cl.³: **A 61 K 35/78, C 07 G 7/00**

(21) Application number: **80902301.3**

(22) Date of filing: **26.11.80**

(86) International application number:
**PCT/JP80/00289**

(87) International publication number:
**WO 81/01517 11.06.81 Gazette 81/14**

(54) PROCESS FOR ISOLATING AND PURIFYING A FRACTION CAPABLE OF INHIBITING MUTAGENICITY OF A MUTAGENIC MATERIAL FROM BURDOCK JUICE.

(30) Priority: **26.11.79 JP 153267/79**

(43) Date of publication of application:
**02.12.81 Bulletin 81/48**

(45) Publication of the grant of the patent:
**04.07.84 Bulletin 84/27**

(84) Designated Contracting States:
**FR**

(56) References cited:
**DE - A - 2 810 293**

**Agricultural and Biological Chemistry Vol. 42, No. 6, Page 1235 to 1238 (1978)**

(73) Proprietor: KANEBO LTD.
17-4 Sumida 5-chome Sumida-ku
Tokyo 131 (JP)

(72) Inventor: MORITA, Kazuyoshi
5-12-13 Kotobuki-cho Odawara-shi
Kanagawa-ken (JP)

(74) Representative: Lepeudry-Gautherat, Thérèse et al, Cabinet Armengaud Jeune Casanova, Akerman, Lepeudry 23 boulevard de Strasbourg
F-75010 Paris (FR)

Courier Press, Leamington Spa, England.

**0 040 641**

### Description

This invention relates to a process for isolating and purifying a des-mutagenic factor contained in burdock juice.

It has become well known recently that practically all of the carginogens exhibit mutagenicity and that the possibility of the mutagens being carcinogens is great. Take, for example, substances that are encountered in our daily life such as AF-2 (2-furyl)-3-(5-nitro-2-furyl)acrylamide, Trp-P-1 (3-amino-1,4-dimethyl-5H-pyrido[4,3-b]indole and Trp-P-2 (3-amino-1-methyl-5H-pyrido[4,3-b]indole), which latter two are present in the scorched portions of say fried, roasted or broiled meat and fish. It is reported that these substances exhibit mutagenicity and are substances that induced carcinogenesis in animals.

We previously suggested a process for isolating and purifying a des-mutagenic factor contained in cabbage juice (see Japanese Laid-Open Patent Publication No. 122715/79 and the corresponding German Patent Application DE 2.810.293. This process involves the steps of:

(1) centrifuging the juice of cabbage,
(2) ultracentrifuging the resulting supernatant,
(3) contacting the supernatant with an anion exchange cellulose,
(4) applying the passed fraction to a column of a cation exchange cellulose,
(5) collecting the active fraction that is eluted at a low salt concentration, and
(6) adsorbing said fraction on a molecular sieve to further purify the fraction.

The final fraction thus obtained exhibits a characteristic absorption spectra at 280 nm and 404 nm and is a hemoprotein that is stable to heat but has the property of being deactivated by the proteolytic enzymes.

This des-mutagenic factor that is obtained by isolation from cabbage juice is known to inhibit not only the mutability of Trp-P-1(3-amino-1,4-dimethyl-5H-pyrido[4,3-b]indole and Trp-P-2(3-amino-1-methyl-5H-pyrido[4,3-b]indole but also that of N-butyl-N-acetoxynitrosoamine, the reaction products of sorbic acid with nitrites, 2-aminoanthracene, ethidium bromide, as well as the various mutagens that manifest mutagenicity as a result of the metabolism of the pyrolysates of such amino acids as tryptophan, ornithine, etc.

The final fraction obtained from cabbage juice has however the drawback that it is deactivated in the copresence of a proteolytic enzyme. Furthermore, it not only cannot inhibit the mutability of those mutagens that manifest mutagenicity without having been metabolized, such as the oxidation colors (e.g. 4-nitro-o-phenylenediamine and 2-nitro-p-phenylenediamine), but rather has the effect of markedly enhancing (i.e. promoting) the mutability.

We have qualitatively reported in the *Agric. Biol. Chem.,* 42 (6), pp. 1236—1238, 1978 that the supernatant obtained by centrifuging the juices of cabbage, brocolli, green pepper, eggplant, apple, burdock, shallot, ginger, pineapple and mint leaf inhibit the mutability of the pyrolysate of tryptophan.

While of all of these vegetables and fruits exhibit the activity of inhibiting the mutability ascribable to those substances that manifest mutagenicity on being metabolized, our further researches have now revealed the fact that of these, only the active fraction obtained from burdock and in the purified state showed superior activity in inhibiting the mutability ascribable to those substances that manifest mutagenicity without having been metabolized, such as the oxidation colors. It is thus seen that the fraction obtained from burdock demonstrates an extremely unique activity.

Further, in accordance with our researches, it has been found that the water-soluble substance obtained in the same manner as in the case with that obtained from burdock juice, from plants of the same composite family as that of burdock, for example, the juice of shungiku, butterbur and lettuce also did not show the activity of inhibiting the mutability ascribable to those substances that manifest mutagenicity without having been metabolized.

In the above-mentioned literature there is only a disclosure of supernatants obtained by centrifuging juices of vegetable and fruits, and there is no mention or suggestions as regards a method of isolating an active component from said supernatant and purifying same. Hence, there is no mention or suggestion of an active component in a purified state.

Disclosure of the Invention

It is therefore an object of this invention to provide in a purified state a des-mutagenic factor that is contained in burdock.

Another object of this invention is to provide in a purified state a des-mutagenic factor that possesses the activity of inhibiting the mutability of those substances that manifest mutagenicity without having been metabolized.

A further object of this invention is to provide in a purified state a des-mutagenic factor which shows no substantial decline in its activity for inhibiting mutability even when it is treated at elevated temperatures or treated with a proteolytic enzyme.

An additional object of this invention is to provide a process by which such a des-mutagenic factor can be efficiently isolated from burdock and be purified.

2

Other objects and advantages of this invention will become apparent from the following description.

The burdock-derived active fraction in a purified state as provided by this invention is a water-soluble substance contained in burdock that is adsorbed on an anion exchange cellulose but not on a cation exchange cellulose. It has an absorption wavelength peak in the range 280 nm—300 nm and an activity of inhibiting mutability.

The des-mutagenic factor provided by this invention can be isolated from burdock and purified by a process comprising the steps of:

(a) centrifuging the juice of burdock to remove foreign matter therefrom,

(b) mixing a phosphate buffer solution with the resulting supernatant followed by salting out the resulting mixture by adding a water-soluble alkali metal salt or ammonium salt of an inorganic acid, and thereafter collecting the precipitate,

(c) dissolving the precipitate in a phosphate buffer solution followed by dialyzing the resulting solution, and thereafter

(d) submitting the dialysis solution to ultrafiltration to withdraw a concentrated solution, which optionally may be lyophilized to yield a powder.

Burdock, as used herein, is the plant *Arctium Lappa Linne* or *Arctium Lappa L.* of the genus *Arctium* of the composite family.

The burdock to be used in this invention for isolation of said active fraction may be not only the roots of burdock but also its fruits, stems and leaves. Hence, it is possible to directly use the harvested burdock without separating it into its parts. Again, it may be used either in its fresh or dried state.

It has been found by our studies that the active fraction obtained from burdock as provided by this invention has such superior properties as that its activity of inhibiting mutability shows no substantial decline even when the fraction is treated at elevated temperatures, say at about 100°C for 15 minutes, or when it is treated with a proteolytic enzyme.

It has also been found that as other characteristics of this active fraction the activity of inhibiting mutability is caused to decline greatly by means of the manganese ions but shows no substantial decline by means of the magnesium or calcium ions.

In carrying out the process of the invention to isolate from burdock the substance that inhibits mutability and purifying same, first, the foreign matter contained in the burdock juice is removed by submitting it to centrifugation.

The burdock juice can be prepared in the following manner. After washing the burdock with water, it is fed to a juicer equipped with a filter to remove a major portion of the fibrous matter to give a fresh juice. Alternatively, a powder of dried burdock can be suspended over a sufficient period of time in water or a phosphate buffer solution at an elevated temperature, as required, or also while being ground down with a triturator. The powder of dried burdock should be one that is as fine as possible. When using this powder, the juice should preferably be prepared by treating the powder at usually about 20° to about 85°C for about 2—24 hours while grinding down the powder with a triturator.

The burdock juice thus prepared is submitted to centrifugation to remove the foreign matter therefrom. The centrifugation may be carried out by the usual centrifugation at 7000—15,000 x G, but it also may be carried out by ultracentrifugation at 100,000—200,000 x G. Again, it is also possible to perform the usual centrifugation followed by ultracentrifugation. The usual centrifugation is usually carried out for 30—60 minutes. The fibrous matter, chloroplast or mitochondria are removed by this centrifugation. On the other hand, the ultracentrifugation is preferably performed for usually 1—2 hours. The microsome or ribosome are removed by this treatment.

To the supernatant obtained after removing the foreign matter from the burdock juice in the matter described hereinabove is then added a phosphate buffer solution. When the juice has been prepared by adding a burdock powder to a phosphate buffer solution, a further addition of a phosphate buffer solution need not be made. It is to be understood that this mode is also included in the scope of this invention.

The phosphate buffer solution used is preferably one whose pH usually ranges from about 6.5 to about 7.5. Further, it is convenient to use as the phosphate buffer solution one whose concentration is usually about 1—2 moles. Such a phosphate buffer solution is preferably added in an amount of 1/20—1/100 parts by volume of the supernatant. Further, the phosphate buffer solution should preferably be added in an amount such that the concentration of the phosphate ions in the supernatant added the phosphate buffer solution is about 10 to about 400 mM.

As the salt to be added to the resulting solution for carrying out its salting out, usable are water-soluble alkali metal salts or ammonium salts of inorganic acids. Examples of inorganic acids are such mineral acids as carbonic acid, sulfuric acid and phosphoric acid. As the alkali metal salts or ammonium salts of such inorganic acids, usable are preferably potassium carbonate, ammonium sulfate, sodium sulfate, potassium phosphate, etc. The water-soluble alkali metal salt or ammonium salt of an inorganic acid is preferably used in an amount of about 30—80% by weight based on the supernatant-phosphate buffer solution mixture.

The thus salted out solution containing a precipitate is then submitted to a treatment to isolate the precipitate therefrom. Various means such as filtration or centrifugation can be performed for accomplishing the isolation of the precipitate. The filtration can be carried out at atmospheric, reduced or superatmospheric pressure. The isolation of the precipitate is however preferably carried out by centrifugation. The centrifugation is carried out under the aforementioned conditions by the usual centrifugation technique.

The precipitate obtained by isolation is then dissolved in a phosphate buffer solution (pH 6.5—7.5) and submitted as a solution to dialysis. The phosphate buffer solution is preferably used in an amount of usually about 5—10 ml per gram of the precipitate. While the dialysis can be carried out against water or a phosphate buffer solution, usually preferred is the use of the latter.

The dialysis solution from which low molecular weight substances have been removed by dialysis is then submitted to ultrafiltration. Concentration of the dialysis solution takes place by ultrafiltration. It is preferred that the ultrafiltration be continued until the volume of the dialysis solution becomes 1/3—1/5 of the original volume. As filters usable in ultrafiltration, mention can be made of say PM-30, XM-50, XM-100 and XM-300 (manufactured by Amicon Far East Ltd.), of which especially preferred is the ultrafiltration membrane XM-300, which passes molecules of molecular weights below about 300,000.

The concentrated solution thus resulting after having been ultrafiltered constitutes the active fraction of this invention. This concentrated solution can also be powderized by the usual lyophilization procedure, as required.

When the concentrated solution is to be submitted to lyophilization, it is preferred that water in an amount of about 3—5-fold volume be added to the solution followed by again submitting the solution to ultrafiltration before carrying out its lyophilization.

The des-mutagenic factor-containing active fraction in the form of a concentrated solution or powder and in a purified state, as provided by this invention, is administered as such to man or animals other than man or after having been suitably combined with other pharmaceutically acceptable carriers or adjuvants.

There is thus provided in accordance with this invention a pharmacuetical composition containing the active fraction of this invention along with a suitable pharmaceutically acceptable carrier or adjuvant, or a medicine containing said composition. As the carriers or adjuvants, included are those which are usually known to those skilled in the art, such as excipients, lubricants, disintegrators, coating agents for capsule, etc.

The medicine may be in the form of say tablets, granules, sugar-coated pills, powder, syrup, solution or capsules.

When the active fraction of this invention is in the forms indicated hereinabove, it is preferably administered orally. The active fraction of the invention can also be administered externally (e.g. application to the skin). In this case, it is preferably used in the form of an ointment, e.g. one which uses an oil-soluble base such as wax, or in the form of a water-containing emulsion or an aqueous solution.

There is provided in accordance with this invention a method of preventing the occurrence of mutations in those animals susceptible to mutagenesis, this being accomplished by administering to such animals a pharmaceuticaly effective amount of the active fraction of this invention, along with pharmaceutically acceptable carriers or adjuvants, as required. The dosage can be suitably determined in accordance with the animal to be treated by such specialists as doctors and pharmacists. It is usually about 100 mg—2.5 g per kg body weight per day.

As can be appreciated from the fact that the active fraction of this invention is a component contained in burdock that is eaten as food, it is practically free of toxicity.

By animals susceptible to mutagenesis are meant to be those animals that have say partaken a mutagen or have come into contact with a mutagen, or are expected to partake a mutagen or come into contact with a mutagen.

The active fraction obtained by lyophilization, as provided by the process of this invention, is conveniently used for preparing any form of medicine. It is a water-soluble and stable brown powder free of odor, and its storage stability is especially good.

According to our studies, it is belived that the des-mutagenic activity of the active fraction of this invention is manifested in such a manner that it inhibits the mutability manifested by the mutagens by acting directly on said mutagens. Hence, the active fraction of this invention is preferably administered preventively to especially those animals that are expected to partake mutagens or those animals which are clearly known to have partaken mutagens.

The following example will serve to more fully illustrate the procedures involved in isolating and purifying the active fraction in accordance with the process of this invention as well as the des-mutagenic activity of the active fraction and its other properties.

## Example

(1) Isolation of the active fraction from burdock and its purification.

Burdock (5000 g) was washed with water and then crushed in a juicer (model JC-540A manfuactured by Toshiba Co.) to obtain about 3400 ml of burdock juice.

This juice (1400 ml) was centrifuged at 9000 × G for 30 minutes to obtain 2200 ml of a clear brown supernatant.

1/20 amount by volume of a 1 M phosphate buffer solution (pH 6.8) was mixed with this supernatant, after which the mixture was salted out by adding to 1100 ml of the mixture 80% by weight of the mixture of ammonium sulfate. The salted out mixture was then submitted to centrifugation at 9000 × G for 15 minutes to yield 80 g of a precipitate.

This precipitate (60 g) was dissolved with a 50 mM phosphate buffer solution (pH 6.8) and made up to 600 ml. The solution was dialyzed at 4°C against the aforesaid phosphate buffer solution (pH 6.8) to give 696 ml of a dialysis solution.

This dialysis solution (464 ml) was then concentrated to 1/3 its original volume by submitting it to ultrafiltration using a membrane filter XM-300 (manufactured by Amicon Far East Ltd.). The concentrated solution was made up to 200 ml by adding a 50 mM phosphate buffer solution (pH 6.8), and it was again concentrated by ultrafiltration using the same filtration as mentioned hereinbefore to give 140 ml of a concentrated solution.

Of the concentrated solution obtained by ultrafiltration, 70 ml was lyophilized at −54°C for 24 hours, using a lyophilizer (Model FDX-1-54 manufactured by Central Chemical Co.) to yield 450 mg of a dry powder. (3) Ultraviolet absorption properties of the active fraction.

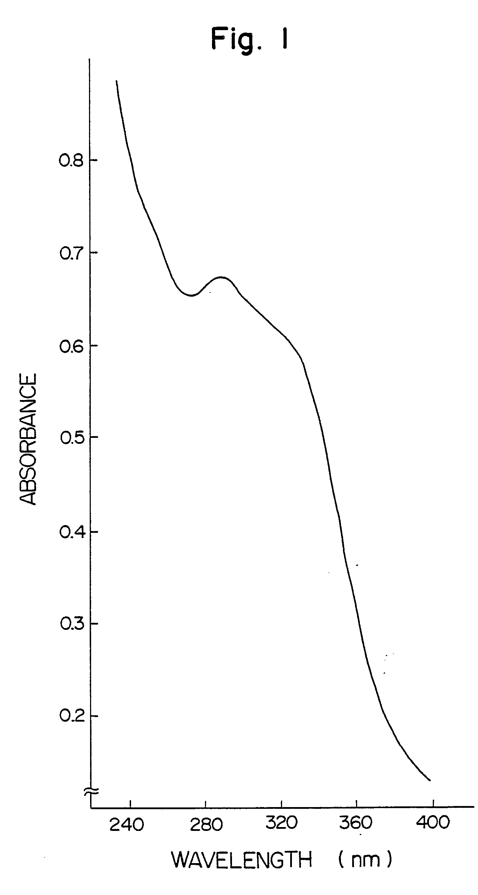

Figure 1 shows an ultraviolet absorption curve as obtained by plotting the measurements made on an aqueous solution of the lyophilized powder. In Figure 1 the absorbance is plotted as ordinate, and the wavelength (nm) is plotted as abscissa. As is apparent from Figure 1, the active fraction of this invention has an absorption wavelength peak in the range 280 nm—300 nm (maximum wavelength about 290 nm).

(4) Absorbance of the active fraction on an anion exchange resin and a cation exchange resin.

The concentrated solution (27 ml) obtained by the method of (1), above, was chromatographed on a column of DEAE-cellulose (an anion exchange cellulose) under the conditions of a column size of 2.5 × 12 cm, elution rate of 36 m/hr, and an amount of eluiton of 10 ml/test tube. The absorbance was examined by performing the elution using as the eluent buffer solution a 50 mM phosphate buffer solution in which had been dissolved potassium chloride with a concentration gradient ranging from 50 mM to 2M. Fraction exhibiting inhibitory activity were not detected in the case of the fractions that were eluted without having been absorbed on the DEAE-cellulose column.

The concentration solution (7 ml) was chromatographed in a similar manner on a column of CM-cellulose (cation exchange cellulose) under the following conditions: column size of 2.5 × 7 cm, elution rate of 40 ml/hr, and amount of elution of 5.8 ml/test tube. The absorbance was examined by performing the elution using as the eluent buffer solution a 50 mM phosphate buffer solution in which had been dissolved potassium chloride with a concentration gradient ranging from 50 mM to 0.1 M.

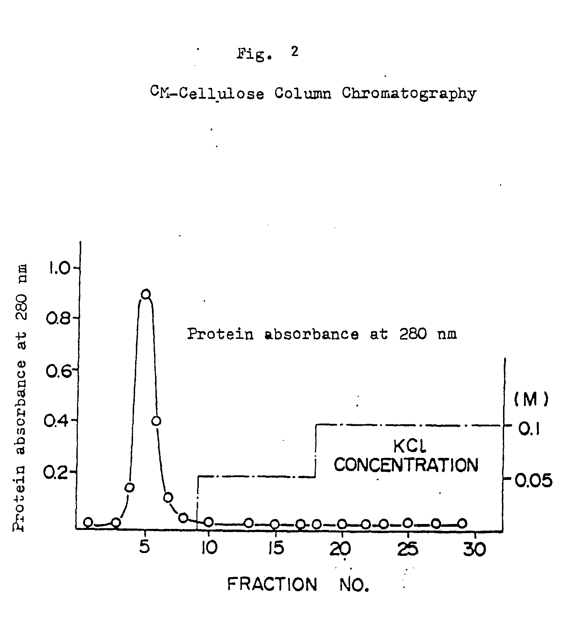

All of the solution was eluted without having been absorbed on the CM-cellulose column. An elution curve such as shown in Figure 2 of the accompanying drawings was obtained. When the eluted fraction was tested for its inhibitory effects by the hereinafter described method, the des-mutagenic rate was 88%. This value was about the same (87.4%) as that of an aqueous solution of a dry powder in an amount of 500 microliters/plate shown in the hereinafter-given Table 2. It is also seen from the results obtained in this test that the active fraction of this invention is a polyelectrolyte possessing a strong anionic base.

(5) Inhibitory effects of the active fraction

(a) The test for determining the inhibitory effects was conducted in the following manner. To a solution of a mutagen in a 0.02 ml of dimethyl sulfoxide was added 0.5 ml of a fraction obtained in each of the steps of the method of (1), above, or the final fraction (the active fraction of this invention), after which the reaction was carried out at 37°C for 30 minutes. As control, the reaction was carried out in the same manner but using 0.5 ml of a phosphate buffer solution instead of the several fractions. The reaction mixtures were then sterilized by heating at 100°C for 10 minutes. After completion of the heat treatment, 3 ml of soft agar (0.6% Difco Agar) and 0.1 ml of a suspension of Salmonella TA98 (histidine requiring His⁻) were added, and the mixtures were poured onto a hereinafter-described selective agar medium. The culture was carried out at 37°C for 2 days, after which the colonies of revertants (histidine non-requiring His⁺) were counted.

In the case the mutagen used was one which manifests mutability only upon being metabolized, such as 2-aminoanthracene, ethidium bromide, Trp-P-1 or Trp-P-2, the test was conducted in the same manner but after the addition to the aforesaid 0.3 ml of soft agar of the S-9 mix prepared as described below. To wit, using SD rat liver, a liver homogenate whose metabolic enzyme activity has been enhanced by PCB is centrifuged to give a liver microsome fraction (S-9). The S-9 mix is then prepared by adding to the liver microsome thus obtained inorganic salts in the following amounts.

Inorganic salts to be added to the S-9 mix:

| | |
|---|---|
| Liver microsome | 3 ml |
| 0.25 M phosphate buffer solution | 4 ml |
| 0.16 M MgCl₂ | 0.5 ml |

| | |
|---|---|
| 0.66 M KCl | 0.5 ml |
| 0.05 M G-6-P | 1.0 ml |
| 0.04 M NADP | 1.0 ml |

Composition of selective agar medium:

| | |
|---|---|
| MM (X20) | 50 ml |
| 40% Glucose | 10 ml |
| 0.8% Difco nutrient broth | 10 ml |
| Biotin (100 $\mu$g/ml) | 1 ml |
| Agar | 15 g |
| Distilled water | 930 ml |

Composition of MM (X20):

| | |
|---|---|
| $(NH_4)_2SO_4$ | 2.0% |
| $KH_2PO_4$ | 20.0% |
| $MgSO_4 \cdot 7H_2O$ | 0.2% |
| Sodium citrate | 1.0% |

PH 7.0 with KOH

The activity of inhibiting the mutability of the mutagens was tested in the manner described hereinabove. Taking the amount (ml) of the fraction required for inhibiting 50% of the mutability, the activity was expressed on the basis of the amount (mg) of protein contained per unit volume (ml) of the fraction.

(b) Inhibition of the mutability of 2-nitro-p-phenylenediamine (80 $\mu$g/plate).

Table 2 shows the influence of concentration on the des-mutagenic effect of the dry powder (active fraction of this invention).

TABLE 1

| Amount of 2-nitro-p-phenylenediamine ($\mu$g/plate) | Amount of aqueous solution of dry powder *1 ($\mu$l/plate) | Des-mutagenic rate *2 (%) |
|---|---|---|
| 80 | 500 | 87.4 |
| 80 | 300 | 85.1 |
| 80 | 100 | 79.4 |
| 80 | 10 | 22.3 |
| 80 | 0 | 0 |

*1: An aqueous solution of dry powder is one in which a lyophilized powder has been dissolved in water.

*2: Des-mutagenic rate (%)

$$= \frac{a - b}{a - c} \times 100$$

6

where

a: Number of colonies of revertants of 2-nitro-p-phenylenediamine treated with a phosphate buffer solution.

b: Number of colonies of revertants of 2-nitro-p-phenylenediamine treated with the active fraction of this invention.

c: Number of colonies of natural revertants.

As can be seen from the results of Table 1, which show the influence of the concentration of the active fraction of this invention on its inhibitory effects, the results are satisfactory when used with 2-nitro-p-phenylenediamine.

Table 3 shows the inhibitory effects of the invention active fraction (final fraction) on various mutagens.

TABLE 3

| Mutagen | Concentration ($\mu$g/plate) | S-9 mix add or not added | Des-mutagenic rate (%) |
|---|---|---|---|
| 2-nitro-p-phenylenediamine | 80 | not added | 89.9 |
| | 80 | added | 92.5 |
| 4-nitro-o-phenylenediamine | 40 | not added | 60.7 |
| | 40 | added | 56.9 |
| ethidium bromide | 10 | do | 96.5 |
| 2-amino-anthracene | 4 | do | 97.8 |
| Trp-P-1 | 0.2 | do | 96.1 |
| Trp-P-2 | 0.2 | do | 95.5 |

The results of Table 3 show the especially remarkable inhibitory effects of the active fraction of this invention. To wit, whereas the inhibitory factor present in the juices of cabbage and brocolli, plants that have been reported in the aforementioned literature, exhibit inhibitory effects when used with only the those mutagens that manifest mutability after having been metabolized (ethidium bromide, 2-aminoanthracene, Trp-P-1 and Trp-P-2), but no inhibitory effects when used with a part of the oxidation colors that manifest mutability without having been metabolized (e.g. 2-nitro-p-phenylenediamine or 4-nitro-o-phenylenediamine), the fraction of this invention demonstrates, as shown in Table 3, remarkable inhibitory effects not only when used with the former class of mutagens but even when used with the latter. Its function and effects are thus exceedingly unique.

(6) Heat resistance of the active fraction

Next, the heat resistance of the final fraction of this invention was examined. Five hundred milliliters of an invention final fraction (an aqueous solution of a lyophilized powder) heated at 100°C for 15 minutes under reflux and cooling and 500 ml of an aqueous solution of said final fraction not heat treated were each mixed with the various mutagens having the concentrations shown in Table 4, following which the mixtures were reacted at 37°C for 30 minutes.

Next, the test for determining the inhibitory effects was conducted as in (5), above. The results obtained are shown in Table 4.

# 0 040 641

## TABLE 4

| Mutagen | Concentration ($\mu$g/plate) | Final fraction (500 $\mu$l/plate) | Des-mutagenic rate (%) |
|---|---|---|---|
| 2-nitro-p-phenylene-diamine | 200 | not heat treated | 88.6 |
| | | heat treated | 86.1 |
| 2-amino-anthracene | 4 | not heat treated | 96.8 |
| | | heat treated | 95.5 |
| ethidium bromide | 10 | not heat treated | 95.5 |
| | | heat treated | 93.5 |
| Trp-P-1 | 0.2 | not heat treated | 96.8 |
| | | heat treated | 96.2 |
| Trp-P-2 | 0.2 | not heat treated | 95.8 |
| | | heat treated | 96.0 |

It has thus been confirmed from these results that the final fraction of this invention being stable to heat is not deactivated thereby and moreover demonstrates satisfactory inhibitory effects.

(7) Stability of the active fraction to proteolytic enzymes

The stability of the final fraction (an aqueous solution of a lyophilized powder) to proteolytic enzymes was also examined. As the proteolytic enzymes, DNase 1 (an enzyme that breaks down DNA), RNase (an enzyme that breaks down RNA), and Pronase (an enzyme that breaks down various proteins, a product of Kaken Chemical Co., Ltd.) were used. These were each added to the final fraction in such an amount that the final concentration would be 20 $\mu$g/ml, and the mixtures were reacted at 37°C for 30 minutes.

These reaction products (500 $\mu$l) were then mixed with the mutagens shown in Table 5 having the concentrations shown therein, and the mixtures were reacted at 37°C for 30 minutes. After completion of the reaction, the test for determining the inhibitory effects was conducted as in (5), above. The results obtained are shown in Table 5.

## TABLE 5

| Mutagen | Concentration ($\mu$g/plate) | Des-mutagenic rate (%)* | | | |
|---|---|---|---|---|---|
| | | A | B | C | D |
| 2-nitro-p-phenylene-diamine | 200 | 86.6 | 84.7 | 83.9 | 87.0 |
| 2-aminoanthracene | 4 | 96.3 | 94.1 | 93.2 | 96.7 |
| ethidium bromide | 10 | 95.1 | 93.1 | 92.0 | 95.1 |
| Trp-P-1 | 0.2 | 96.7 | 94.6 | 93.7 | 97.1 |
| Trp-P-2 | 0.2 | 95.2 | 97.3 | 91.8 | 95.6 |

*A: When the mutagen is one treated with the final fraction.
B: When the mutagen is one treated with a final fraction that has been treated with DNase 1.
C: When the mutagen is one treated with a final fraction that has been treated with RNase.
D: When the mutagen is one treated with a final fraction that has been treated with Pronase.

These results confirm the fact that the final fraction of this invention being extremely stable to the proteolytic enzymes is not deactivated thereby and moreover manifests satisfactory inhibitory effects.

8

(8) Stability of the active fraction to polyvalent metal ions or protein denaturants

The stability of the final fraction of this invention to the various polyvalent metal ions or protein denaturants was also examined.

Magnesium chloride, manganese chloride and calcium chloride were each mixed and dissolved such that the concentration (final concentration) in the resulting solution would be 10 mM of the invention final fraction (an aqueous solution of a lyophilized powder). On the other hand, in the case of EDTA, a protein denaturant, the mixture and solution thereof was carried out such that its concentration would be 2.5 mM, while in the case of SDS (sodium dodecylbenzenesulfonate), likewise a protein denaturant, the mixture and solution was carried out such that its concentration would be 0.1%. The several solutions were then processed at 37°C for 30 minutes.

Subsequently, the reaction mixtures (500 $\mu$l) were mixed with the mutagens shown in Table 6 having the concentrations indicated therein, following which the reaction was carried out at 37°C for 30 minutes. After completion of the reaction, the reaction products were tested for their inhibitory effects as in (5), above. The results obtained are shown in Table 6.

## TABLE 6

| Mutagen | Concentration ($\mu$g/plate) | Des-mutagenic rate (%)* | | | | | |
|---|---|---|---|---|---|---|---|
| | | A | Mg$^{++}$ | Mn$^{++}$ | Ca$^{++}$ | EDTA | SDS |
| 2-nitro-p-pheny-lenediamine | 200 | 82.8 | 84.9 | 12.0 | 80.3 | 84.0 | 82.3 |
| 2-amino-anthracene | 4 | 95.2 | 94.1 | 8.5 | 90.1 | 92.0 | 94.5 |
| ethidium bromide | 10 | 93.8 | 92.1 | 10.2 | 94.1 | 93.2 | 93.5 |
| Trp-P-1 | 0.2 | 95.3 | 96.7 | 9.8 | 93.0 | 92.3 | 96.0 |
| Trp-P-2 | 0.2 | 96.2 | 95.1 | 8.7 | 94.0 | 95.7 | 93.2 |

*A: When the mutagen used was one treated with the final fraction.

Mg$^{++}$: When the mutagen used was one treated with a final fraction treated with magnesium chloride.

Mn$^{++}$: When the mutagen used was one treated with a final fraction treated with manganese chloride.

Ca$^{++}$: When the mutagen used was one treated with a final fraction treated with calcium chloride.

EDTA: When the mutagen used was one treated with a final fraction treated with EDTA.

SDS: When the mutagen used was one treated with a final fraction treated with SDS.

These results thus confirm the fact that the final fraction of this invention possesses a unique property in that while it is extremely stable to protein denaturants and magnesium chloride and calcium chloride, it shows a great decline in its des-mutagenic activity by means of manganese chloride.

The final fraction of this invention, as is apparent from the various results shown hereinabove, is thus a polyelectrolyte showing an absorption wavelength peak in the range 280 nm — 300 nm and having a strong anionic base. It is an anti-mutagenic substance of high inhibitory activity, which inhibits the mutability of many mutagens, and especially those mutagens that manifest mutability without having been metabolized, the inhibitory activity of which while stable to heat and showing no substantial decline by means of a proteolytic agent is caused to decline greatly by means of the manganese ions.

## Claims

1. A process comprising the steps of:

(a) centrifuging the juice of burdock to remove foreign matter therefrom.

(b) mixing a phosphate buffer solution with the resulting supernatant, followed by salting out the resulting mixture by adding a water-soluble alkali metal salt or ammonium salt of an inorganic acid and thereafter collecting a precipitate,

(c) dissolving the precipitate in a phosphate buffer solution followed by dialyzing the resulting solution, and thereafter

(d) submitting the dialysis solution to ultrafiltration to withdraw a concentrated solution followed by optionally lyophilizing the concentrated solution to powderize same thereby isolating and purifying and desmutagenic factor having an absorption wavelength peak in the range 280 nm—300 nm, said desmutagenic factor being a substance that is absorbed on an anionic exchange cellulose but not on a cationic exchange cellulose, the activity of inhibiting mutations of said desmutagenic factor being caused to decline greatly by means of manganese ions but showing no substantial decline by heating at 100°C for 15 minutes.

2. The process as defined in claim 1 which comprises mixing said phosphate buffer solution in an amount of 1/20—1/100 by volume of the supernatant resulting after removed foreign matter from the burdock juice by centrifugation.

3. The process as defined in claim 1 wherein said water-soluble ammonium salt of an inorganic acid is ammonium sulfate.

4. The process as defined in claim 1 which comprises adding said water-soluble alkali metal salt or ammonium salt of an inorganic acid in an amount of about 30—80% by weight based on the weight of the mixture of the supernatant and the phosphate buffer solution.

5. The process as defined in claim 1 which comprises dissolving the collected salted out precipitate in the phosphate buffer solution in a proportion of 5—10 ml of the latter per gram of the former.

6. The process as defined in claim 1 which comprises carrying out the dialysis against a phosphate buffer solution.

7. The process as defined in claim 1 which comprises concentrating the dialysis solution to 1/3—1/5 of its original volume by ultrafiltration.

8. An active fraction obtained from burdock having the activity of inhibiting mutability obtained by the process as defined in claim 1.

**Patentansprüche**

1. Verfahren, gekennzeichnet durch folgende Stufen:
(a) Zentrifugieren von Klettensaft zur Entfernung von Fremdverunreinigungen,
(b) Mischen einer Phosphatpufferlösung mit der gebildeten überstehenden Lösung und anschliessendes Aussalzen der gebildeten Mischung durch Zugabe eines wasserlöslichen Alkalisalzes oder Ammoniumsalzes einer anorganischen Säure und anschliessendes Sammeln eines Niederschlags,
(c) Auflösen des Niederschlags in einer Phosphatpufferlösung und anschliessendes Dialysieren der gebildeten Lösung und anschliessendes
(d) Unterwerfen der Dialyselösung einer Ultrafiltration unter Abziehen einer konzentrierten Lösung und gegebenenfalls anschliessendem Lyophilisieren der konzentrierten Lösung unter Pulverisierung derselben, wodurch man einen demutagenen Faktor mit einem Absorptionswellenlängenpeak im Bereich von 280 mit 300 nm isoliert und reinigt und wobei der demutagene Faktor eine Substanz ist, die auf einer anionischen Austauschcellulose, nicht jedoch auf einer kationischen Austauschcellulose, absorbiert wird, wobei die Aktivität zum Inhibieren von Mutationen des demutagenen Faktors erheblich durch Manganionen erniedrigt wird, aber keine wesentliche Erniedrigung beim Erhitzen auf 100°C während 15 Minuten vorliegt.

2. Verfahren gemäss Anspruch 1, dadurch gekennzeichnet, dass man die Phosphatpufferlösung in einer Menge von 1/20 bis 1/100 des Volumens der überstehenden Flüssigkeit, die nach der Entfernung der Fremdstoffe von dem Klettensaft nach dem Zentrifugieren zurückbleibt, vermischt.

3. Verfahren gemäss Anspruch 1, dadurch gekennzeichnet, dass das wasserlösliche Ammoniumsalz einer anorganischen Säure Ammoniumsulfat ist.

4. Verfahren gemäss Anspruch 1, dadurch gekennzeichnet, dass man das wasserlösliche Alkalisalz oder Ammoniumsalz einer anorganischen Säure in einer Menge von etwa 30 bis 80 Gew.%, bezogen auf das Gewicht der Mischung, aus der überstehenden Flüssigkeit und der Phosphatpufferlösung, zugibt.

5. Verfahren gemäss Anspruch 1, dadurch gekennzeichnet, dass man den gesammelten ausgesalzenen Niederschlag in der Phosphatpufferlösung in einem Verhältnis von 5 bis 10 ml der letzteren pro Gramm der ersteren auflöst.

6. Verfahren gemäss Anspruch 1, dadurch gekennzeichnet, dass man die Dialyse gegen eine Phosphatpufferlösung vornimmt.

7. Verfahren gemäss Anspruch 1, dadurch gekennzeichnet, dass man die Dialyselösung auf 1/3 bis 1/5 des Ursprungsvolumens durch Ultrafiltration konzentriert.

8. Eine aus Kletten erhaltene aktive Fraktion, die eine Aktivität, die Mutabilität zu inhibieren, aufweist, erhalten nach dem Verfahren gemäss Anspruch 1.

**Revendications**

1. Un procédé comprenant les opérations suivantes:
(a) on centrifuge du jus de bardane pour en éliminer les matières étrangères,
(b) on mélange une solution de tampon au phosphate avec le surnageant obtenu puis on relargue

# 0 040 641

le mélange en lui ajoutant un sel de métal alcalin ou d'ammonium hydrosoluble d'un acide minéral et on recueille le précipité formé,

(c) on dissout ce précipité dans une solution de tampon au phosphate et on dialyse la solution puis

(d) on soumet la solution après la dialyse à une ultrafiltration pour obtenir une solution concentrée, que l'on peut éventuellement lyophiliser pour obtenir une poudre, isolant et purifiant ainsi un facteur antimutagène ayant un pic de longueur d'onde d'absorption situé entre 280 et 300 nm, ce facteur antimutagène étant une substance qui est absorbée sur une cellulose d'échange anionique mais no sur une cellulose d'échange cationique, et l'action inhibitrice de ce facteur sur les mutations étant fortement abaissée par les ions manganèse mais pratiquement pas à la suite d'un chauffage de 15 minutes à 100°C.

2. Procédé selon la revendication 1 dans lequel on mélange la solution de tampon au phosphate dans une proportion comprise entre 1/20 et 1/00 en volume du surnageant résultant de l'élimination par centrifugation des matières étrangères du jus de bardane.

3. Procédé selon la revendication 1 ou 2 dans lequel le sel d'ammonium hydrosoluble de l'acide minéral est le sulfate d'ammonium.

4. Procédé selon l'une quelconque des revendications 1 à 3 dans lequel on ajoute le sel de métal alcalin ou d'ammonium hydrosoluble de l'acide minéral dans une proportion d'environ 30 à 80% du poids du mélange du surnageant et de la solution de tampon au phosphate.

5. Procédé selon l'une quelconque des revendications 1 à 4 dans lequel on dissout le précipité relargué recueilli dans la solution de tampon au phosphate dans une proportion de 1 gramme du précipité pour 5 à 10 ml de la solution.

6. Procédé selon l'une quelconque des revendications 1 à 5 dans lequel on effectue la dialyse contre une solution de tampon au phosphate.

7. Procédé selon l'une quelconque des revendications 1 à 6 dans lequel on concentre la solution après la dialyse, par ultrafiltration, entre le tiers et le cinquième de son volume initial.

8. Fraction de bardane inhibant les mutations, qui a été obtenue par un procédé selon l'une quelconque des revendications précédentes.

# Fig. 1

Fig. 2

CM-Cellulose Column Chromatography